# EUROPEAN PATENT SPECIFICATION

(11) **EP 0 589 636 B1**
(45) Date of publication and mention of the grant of the patent: **02.08.2000**
(21) Application number: 93307372.8
(22) Date of filing: 17.09.1993
(51) Int. Cl.: B03C 1/035, G01N 35/00

(54) **Magnetic separation method and apparatus therefor**
Verfahren und Vorrichtung zur magnetischen Abscheidung
Méthode et appratus pour la séparation magnétique

(30) Priority: 24.09.1992 EP 92308727
(43) Date of publication of application: 30.03.1994
(73) Proprietor: Amersham Pharmacia Biotech UK Limited, Little Chalfont, Buckinghamshire HP7 9NA (GB)
(72) Inventor: Howe, Roland Paul, Calvert, Bucks. MK18 2HQ (GB); Reeve, Michael Alan, Henley-on-Thames, Oxon R69 1TE (GB); Bischof, Daniel, CH-8645 Jona (CH)
(74) Representative: Wilkinson, Stephen John

(56) References cited:
- EP-A- 0 358 948
- EP-A- 0 479 448
- WO-A-91/09308
- US-A- 5 053 344
- INDUSTRIAL & ENGINEERING CHEMISTRY RESEARCH vol. 28, no. 6 , June 1989 , WASHINGTON US pages 803 - 805 S.KIMURA ET AL

## Description

### 1) INTRODUCTION

Magnetic separation techniques are increasingly used for the purification or quantification of biological molecules. Techniques that use magnetically attractable particles for the separation of specific molecules from a liquid are well documented in biochemical, biomedical and molecular biology research.

These techniques involve the suspension of magnetically attractable particles in a liquid that contains molecules of interest in an impure or dilute form. The molecules are then captured by the magnetic particles by virtue of specific, or non-specific interactions. Application of a magnetic field to the vessel containing the particles will cause them to migrate towards the source of the field, thus concentrating them at the wall of the vessel. With the magnetic field still applied the remaining liquid (the supernatant) can be discarded by pouring it off or by using a pipetting device, leaving the pellet of particles intact. Additional liquid can then be added and the magnetic field removed, thus allowing the particles to be resuspended. If the interaction between particle and molecule of interest is disrupted at this stage, the purified/concentrated molecules can be recovered by reapplying the field and removing and retaining the supernatant.

The sequence of manipulations consisting of magnetic separation, supernatant removal , addition of liquid and pellet resuspension is common to most of the techniques that utilise magnetically attractable particles. This process may be repeated a number of times during a typical technique, as there may be an initial capture step, followed by one or more treatment or washing steps and a final recovery step.

Magnetic separation techniques are used routinely in many manually operated procedures, but the principle also has great potential for use in automated or robotic systems. However, the apparatus and manipulations currently used are not amenable to simple and reliable automation for the reasons described in section 2).

This invention, described in section 3), provides systems of apparatus and procedures that enable efficient and relatively simple automation of magnetic separation techniques. The same systems may also be used in manual techniques.

### 2) CONVENTIONAL SYSTEMS.

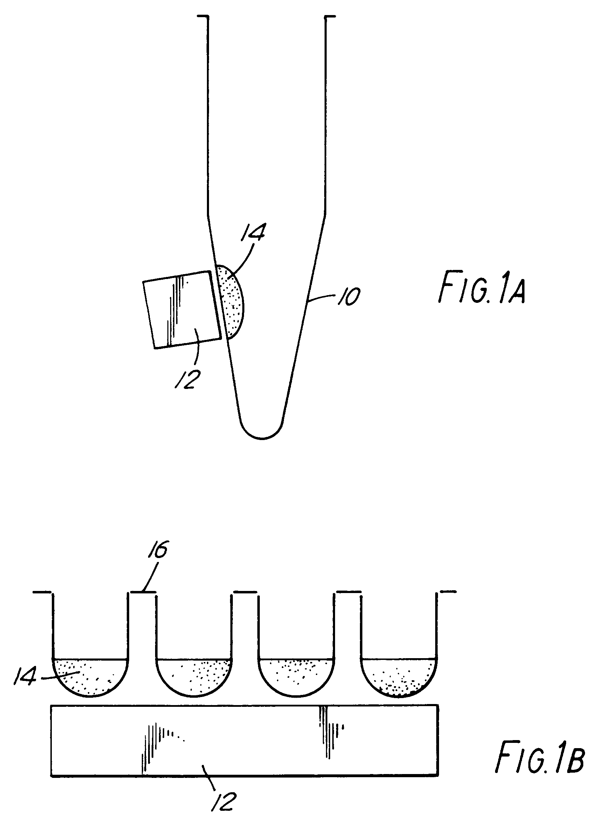

The apparatus typically used to carry out this process consists of a rack into which one or more small volume vessels can be inserted (typically small centrifuge tubes or multi-well plates). An arrangement of dipole magnets allows the magnetic particles to be drawn to the side or bottom of each tube or well. Figure 1 comprises sectional side elevations A and B which illustrate the principles employed by the two most commonly used types of apparatus.

Application and removal of the magnetic field in manual systems is usually achieved by inserting the tubes/plates into the apparatus and then removing them. Automated systems could potentially use mechanically actuated permanent magnets or switched electromagnets, although no such systems are yet commercially available.

In Figure 1A, a single vessel 10, commonly an Eppendorf tube, has been brought into proximity with a magnet 12, whereby a pellet 14 comprising magnetically attractable particles has been formed. In Figure 1B, a microtitre plate 16 has been brought into proximity with a magnet 12, and a pellet 14 comprising magnetically attractable particles has formed in the bottom of each vessel of the plate.

System A uses a magnet which draws the magnetically attractable particles to the side of the vessel, leaving the bottom clear. This enables liquid to drain from the pellet during supernatant removal and be efficiently removed manually or robotically using a pipetting device inserted to the full depth of the vessel. One drawback in automated systems occurs when the pellet must be resuspended in a small volume of liquid, as is usually necessary in order to achieve the maximum possible concentration of the molecule of interest. The pellet adhering to the side of the tube must be washed off by repeatedly drawing up liquid from the bottom of the tube and expelling it at the side of the vessel above the pellet until all the material is resuspended. This process requires accurate control of pipette movement in three dimensions and visual feedback to ensure resuspension, which can not be achieved in a simple robotic system.

It might appear possible to position the magnet 12 nearer the bottom end of the tube 10 so as to form the pellet 14 at a position where it would be immersed by the re-suspending liquid. This is often unsatisfactory in practice, because the magnetic field is then too weak to rapidly draw down magnetically attractable particles that were initially near the surface of the liquid suspension. Also the pellet will still occupy a fairly large vertical segment of the tube, so requiring a large volume of the re-suspending liquid to immerse it.

In EPA 479,448 and also EPA 317,286 are described magnet systems designed to draw down an annular or part-annular pellet of magnetisable beads. This arrangement simplifies the removal of supernatant liquid by a pipette, but the efficient re-suspension of the magnetisable beads remains problematic.

System B illustrates apparatus in which the particles are drawn to the bottom of the vessel. In this case complete resuspension can be easily achieved in manual or automated systems with a simple pipetting device inserted to the full depth of the vessel. Supernatant removal can be performed by either inverting the apparatus or inserting a pipette as close as possible to the pellet. Neither of these methods is very suitable for automation. Inversion is inelegant and complex to achieve in an automated system and is also unacceptable if hazardous materials are involved. Pipetting does not drain the pellet well and again requires visual feedback to position the pipette accurately.

In EP 358 948, magnetic particles used as solid support are placed-in reaction vessels and stepped sequentially past successive magnets for separation.

### 3) THE INVENTION

This invention is based on the idea of using a magnet system which operates in two modes depending on the relative positions of the vessel and the magnet:
- an annular separation position, and
- a pull-down separation position.

A feature which characterizes the invention over EPA 479448 is the use of these two modes in particular the pull-down position.

A preferred magnet system, for use in the method and apparatus aspects of the invention, is described and forms a further aspect of the invention.

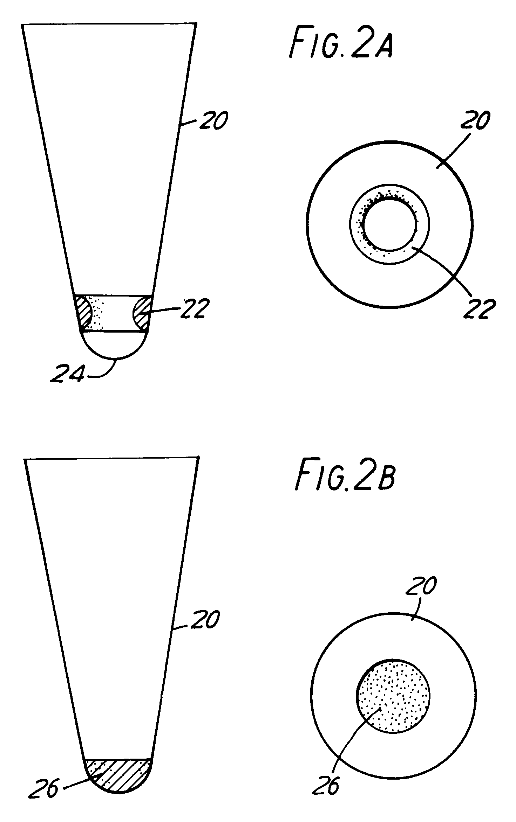

In the annular separation position, the magnet system is arranged to attract the magnetically attractable particles into an annular (ring) or part-annular pellet near the bottom end of the vessel. This conformation enables a pipette, robotic or manual, to be inserted through the centre of the annulus to the bottom of the vessel to fully drain the pellet and remove the supernatant. The top half of Figure 2 of the accompanying drawings, entitled A, annular separation, contains schematic sectional side elevation and plan views of tube 20 with an annular pellet 22 positioned near the bottom end 24.

After removal of the magnetic field, the pellet can be resuspended by adding liquid to the level of the upper edge of the annulus 22 and drawing the liquid into the pipette and expelling it again a number of times. This resuspension can be made more efficient and reliable by two modifications:-
a) After adding the resuspension liquid, drawing the annular pellet down to the bottom of the vessel. This is shown in the lower part of Figure 2, B, pull-down separation, which comprises sectional side elevation and plan views of the same tube with a pellet 26 at the bottom.
b) Positioning the pipette tip a short distance above the bottom of the vessel.

These two modifications cause all of the pellet material to be within the region of maximum flow rate close to the pipette tip, so improving the resuspension efficiency.

Because this system requires only a single dimension of pipette movement (vertical) and visual feedback is unnecessary, automation of the necessary pipette actions is simple and reliable. Although the primary advantages of the system are seen in automated systems, the same system may be used manually.

Thus the invention provides in one aspect a magnetic separation method as claim 1.

In another aspect, the invention provides apparatus which comprises:
an array of vessels (6, 7) each to contain a liquid suspension of magnetically attractable particles;
a pipetting system for transferring liquid to and from the array of vessels;
and a magnet system; and
means for movably mounting the magnet system relative to the array of vessels,
   wherein the magnet system comprises: a back flow plate (2); formed of magnetisable material; a plurality of pairs of permanent magnets (3) each having opposed first and second ends, the first end of each said magnet being positioned on the back flow plate with a N-S axis oriented perpendicular to the back flow plate, the polarity of each permanent magnet of a pair being opposite to that of its neighbour, the permanent magnets of a pair being spaced apart to define between them a work station,
   wherein there is provided means for effecting vertical movement of the magnet system relative to the array of vessels said means being adapted to bring the magnet system to any one of three positions and hold the magnet system in that position, the positions being:
   i) engaged with the array of vessels in an annular separation position;
   ii) free of the array of vessels; and
   iii) engaged with the array of vessels in a pull-down position.

An annular (or part-annular) pellet has the advantage that the particulate material is spread around the wall of the tube rather than up the wall. It is this feature as much as anything that enables the pellet to be positioned so low in the tube as to be immersed in a small volume of liquid added to the tube to resuspend the particles.

An annular or part-annular pellet is one which is positioned on an internal side wall of the vessel (which is here presumed circular although this would not necessarily be so in practice) and extends all or part of the way round the side wall. As noted below, a part-annular pellet may comprise two portions generally on opposite sides of the vessel. Because the pellet is not at the bottom end of the vessel, a pipette can be inserted into the bottom end of the vessel in order to remove supernatant liquid without disturbing the pellet. Because the pellet is close to the bottom end of the vessel, it is immersed in the small volume of fresh liquid used to re-suspend the magnetically attractable particles.

The invention is applicable to round-bottom and conical-bottom vessels generally, of which microcentrifuge tubes (e.g. Eppendorf tubes and Sarstedt tubes) and microtitre plates are examples.

WO 91/12079 describes a method of recovering a biopolymer such as a nucleic acid from solution, by the use of magnetically attractable beads which do not specifically bind the polymer. The beads are suspended in the solution. Then the polymer is precipitated out of solution and becomes non-specifically associated with the beads by bead entrapment. When the beads are magnetically drawn down, the polymer is drawn down with them. After removal of supernatant liquid, fresh liquid can be added to the tubes to resolubilise the polymer and separate it from the beads.

Another standard system makes use of magnetically attractable beads which are coated with a reagent to specifically bind with the polymer to be recovered. The beads are added to a solution of the polymer. A magnet is used to pull the beads, with polymer bound to them, down into a pellet. The supernatant liquid is removed and fresh liquid added. The magnetisable beads are again drawn down. Then the fresh liquid is agitated to re-suspend the magnetisable beads and release into solution the polymer that was associated with them. Finally, the beads are again drawn down and removed.

The magnet system of this invention, involving an annular separation position and a pull-down separation position, is applicable to both these known techniques. The nature of the polymer and of the starting solution is not material to the invention, and a number of these can be listed by way of example:-
a) Alcohol precipitation of nucleic acid molecules from solution;
b) Precipitation of bacteriophage and other viruses from solution;
c) Removal of bacterial DNA, proteins and membranes from bacterial lysates;
d) DNA preparation from bacteriophage or other viruses;
e) Precipitation of bacteria from solution.

The nature of the magnetically attractable particles is not material to the invention. Paramagnetic beads are commercially available and are suitable.

Preferred magnet systems involve permanent magnets, for example of Nd, Fe, B. Alternatively, electromagnet systems may be used. Electromagnet systems that are capable of forming the required fields have been designed and constructed. These multi-coil systems are designed to perform the annular and pull-down separation modes by switching between different coils.

### Drawings

Reference is directed to the accompanying drawings in which:-
Figure 1 comprises sectional side elevations A and B of two conventional magnetic separation systems.
Figure 2 comprises two parts A and B, each of which comprises two drawings. One is a side view and the other is a corresponding top view. Figure 2A illustrates the annular separation step, and Figure 2B illustrates the pull-down separation step of the present invention.
Figure 3A comprises a side view and a corresponding top view of a magnet system for use in the invention.
Figure 3B comprises a side view and a corresponding top view of another magnet system for use in the invention.
Figure 4A is a side elevation of a preferred magnet system according to the invention, and
Figure 4B is an enlargement of part of Figure 4a showing the magnetic field lines.

### MAGNET SYSTEMS

The magnetic fields required to perform the annular and pull-down separations can be achieved in a number of ways, and some of these are described below.

In one embodiment, the magnet system is arranged with a N-S axis vertical or at least having a vertical component. Thus the magnet system may comprise a single magnet combined with an open-top cylindrical magnetic flux conductor. The bottom end of the vessel containing the liquid suspension of magnetically attractable particles is inserted into the open top of the cylindrical magnetic flux conductor in order to perform an annular pellet comprising the magnetically attractable particles.

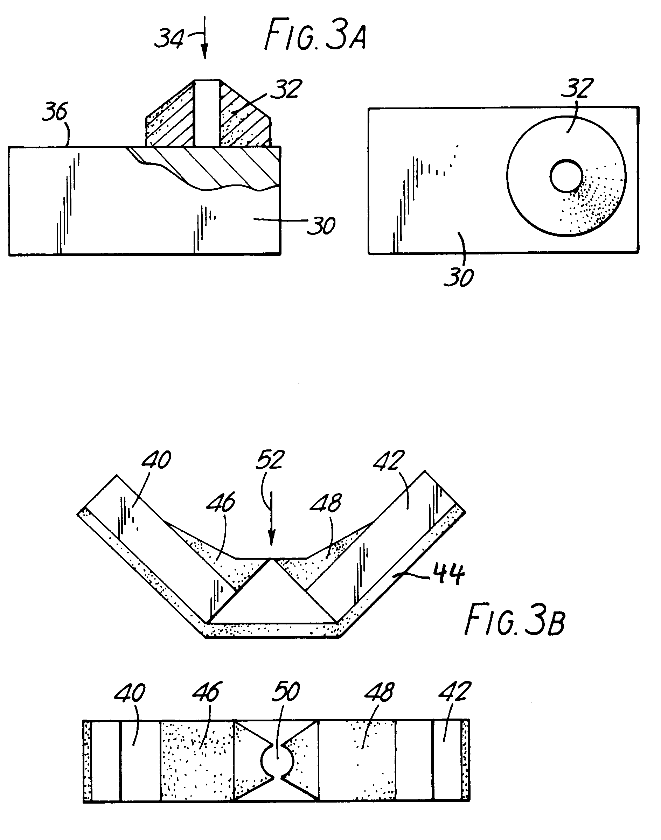

Figure 3A comprises a side view and a corresponding top view of an embodiment of this system. The magnet system comprises a permanent magnet 30 of Neodymium/Iron/Boron, combined with an open-top cylindrical iron magnetic flux conductor 32. In use, a vessel containing a liquid suspension of magnetically attractable particles is lowered in the direction of the arrow 34 until its bottom end rests on/in the open top of the cylindrical magnetic flux conductor 32. There is a node of zero magnetic field along the vertical axis of the tube. By this means, an annular pellet is drawn down as shown in Figure 2A. A pipette is inserted into the bottom end 24 of the tube, the supernatant liquid is removed, and the magnet is taken away. Then fresh liquid is introduced and the annular pellet resuspended in it. The pull-down separation (Figure 2B) can conveniently be achieved by bringing the bottom end 24 of the vessel close to the flat surface 36 of the magnet. In an automated system it is likely to be more convenient to move the magnet system up underneath the vessel rather than to move the vessel itself.

Alternatively, the magnet system may generate a magnetic field gradient having a vertical component in a region into which the vessel containing the liquid suspension of magnetically attractable particles is brought in proximity with the magnet system. An embodiment is shown in Figure 3B which comprises a side view and corresponding top view of the magnet system. This comprises a pair of permanent magnets 40, 42 on opposite sides of the position to be occupied by the vessel containing the liquid suspension. Each magnet is inclined with a lower pole facing an opposite lower pole of the other magnet. Thus for example, the N-S axis of the magnet 40 goes from 2 o'clock to 8 o'clock in the side view, and the N-S axis of the magnet 42 correspondingly goes from 10 o'clock to 4 o'clock. A lower magnetic flux conductor 44 overlies and is bonded to the lower side face of each magnet and serves both to conduct the field between the two magnets and to connect the two magnets together to form a rigid structure.

The upper side surface of each magnet 40, 42 carries an overlying upper magnetic flux conductor 46, 48. These two upper magnetic flux conductors define between them an opening 50 into which the bottom end of a vessel containing a liquid suspension of magnetically attractable particles is inserted, as shown by the arrow 52, in order to form part-annular pellets comprising the magnetically attractable particles.

The gradient field magnet system of Figure 3B is somewhat more complex than the open field magnet system of Figure 3A, but gives faster separation for a given mass of magnet.

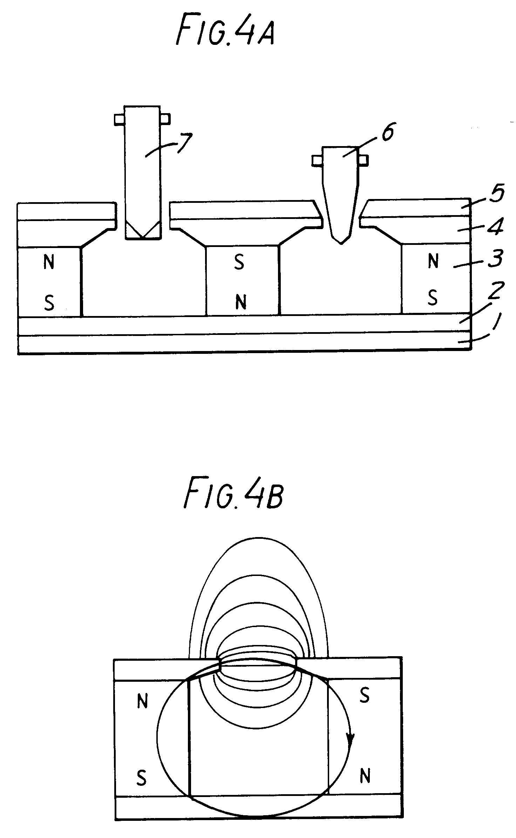

A preferred magnet system is shown in Figures 4a and 4b. The magnet system comprises a carrier plate 1 of aluminium; a backflow plate 2 of iron overlying the carrier plate; a row of permanent magnets 3 of Nd, Fe, B; a field concentrator plate 4 of iron overlying the permanent magnets; and a cover plate 5 of aluminium overlying the field concentrator plate. Each permanent magnet is positioned on the back flow plate with its N-S axis perpendicular to the back flow plate, the polarity of each permanent magnet being opposite to that of its neighbour or neighbours. The permanent magnets are spaced apart at regular intervals to define between them work stations, of which two are shown in Figure 4a. A hole is provided through the field concentrator plate and the overlying cover plate for locating a vessel at each work station. A Sarstedt tube 6 is shown in position at one work station, and an Elkay tube 7 in position at the other.

The field concentrator plate 4 has a flat upper surface and a tapered cross section. The plate is thicker where it overlies each magnet and thinner adjacent each work station. This tapered cross section creates a stray flux from magnetic north to south. Annular and pull-down separation positions are defined by the insertion depth of the vessel into the field concentrators. The intensity and the deep action of the stray field is mostly determined by the shape and material of the field concentrators. The other parameters affecting the magnetic field are the shape and material of the permanent magnets.

If the magnets are positioned in a single row, then the number of magnets is one more than the number of work stations, and each work station is affected by two magnets. Alternatively, the magnets may be arranged in an array with e.g. 4 or 6 magnets surrounding each work station.

### EXAMPLE 1

Two prototype instruments are working routinely in Applicant's laboratory to execute complete DNA preparation procedures using magnetic particles. These use an array of 48 permanent magnets with cylindrical flux conductors of the type shown in figure 3A. These are mounted on a motorised table which can move in the X and Z axes. This assembly is situated below a fixed 48 tube rack, above which is a robotic pipetting system which has movement in all three axes. The pipetting system consists of two syringe pumps connected to a needle mounted on the X/Y/Z robot via plastic tubing and a T union. All systems are controlled from a single computer which enables complete procedures to be executed unattended.

Annular separation is achieved by moving the magnet table such that the base of each tube locates in the corresponding flux conductor. Moving the table to bring the base of the tube in contact with the upper surface of the magnet (to the left of the flux conductor in this case) produces the pull-down separation. The table can also be moved down sufficiently far that any field effect on the tubes is negligible.

The tube rack could potentially be moved to a fixed array of magnets instead, but this complicates the process of moving the pipetting needle to the tubes and so is not so suitable for an automated system.

### EXAMPLE 2

The magnet system described in Figure 4 has been used with two different kinds of tubes, both widely available commercially: Sarstedt 1.5 ml propylene tubes; and Elkay 5 ml propylene tubes. Polymer concentrations in solution in the vessels have ranged from 0.1 mg/ml to 5 mg/ml. Solvents have included water, ethanol, 70% ethanol in water, isopropanol, 20% polyethylene glycol in water, and neutralised E. coli lysates. Solution volumes have ranged from 20 to 1400 µl in Sarstedt tubes and from 100 -5000 µl in Elkay tubes. The following protocols have been established:-
a) For Elkay tubes containing 2-5 ml of solution. For annular separation, insert the tube 15 mm into the field concentrator; allow 90 seconds settling time; slow up movement to an insertion depth of 6 mm. The insertion depth is the distance from the bottom of the tube up to the top surface of the field concentrator plate. For pull-down separation, the protocol is: initial insertion depth 15 mm; settling time 90 seconds; slow up movement to an insertion depth of 1 mm.
b) Elkay tubes containing 100 µl to 2 ml solution. For annular separation, the insertion depth is 6 mm. For pull-down separation, the insertion depth is 1 mm. In each case a settling time of 90 seconds is allowed.
c) Sarstedt tubes. For annular separation the insertion depth is 4 mm. For pull-down separation, the insertion depth is 1 mm. Again, the settling time is 90 seconds.

## Claims

1. A magnetic separation method which comprises the steps of:
a) bringing a vessel having a bottom end and containing a liquid suspension of magnetically attractable particles into proximity with a magnet system, by effecting vertical movement of the magnet system relative to the vessel, whereby there is formed an annular or part-annular pellet comprising the magnetically attractable particles close to but not at the bottom end of the vessel,
b) removing supernatant liquid from the vessel, and
c) adding fresh liquid to the vessel;
d) moving the same magnet system to a draw down position and holding it in that position so as to draw the magnetically attractable particles to the bottom of the vessel;
e) removing the magnet system;
f) and re-suspending the magnetically attractable particles in the fresh liquid.

2. A method according to claim 1 applied to the treatment of a solution of a polymer in a vessel having a bottom end, by the use of magnetically attractable particles which do not specifically bind the polymer, comprising the steps of:
- suspending the magnetically attractable particles in the solution so as to form a liquid suspension of the magnetically attractable particles,
- precipitating the polymer out of solution whereby it becomes non-specifically associated with the magnetically attractable particles,
- performing steps a, b) and c) of claim 1,
- and re-dissolving the polymer in the fresh liquid.

3. A method as claimed in claim 2, wherein the polymer is a nucleic acid.

4. A method as claimed in any one of claims 1 to 3, wherein the magnet system comprises a single magnet (30), combined with an open-top cylindrical magnetic flux conductor (32), and the bottom end of vessel containing the liquid suspension of magnetically attractable particles is inserted into the open top of the cylindrical magnetic flux conductor in order to form an annular pellet comprising the magnetically attractable particles.

5. A method as claimed in any one of claims 1 to 3, wherein the magnet system comprises a pair of magnets (40, 42) on opposite sides of the (position to be occupied by the) vessel containing the liquid suspension, each magnet being inclined with a lower pole facing an opposite lower pole of the other magnet, the lower side faces of the magnets being connected together by a lower magnetic flux conductor, and wherein the upper side face of each magnet carries an upper magnetic flux conductor (46, 48), the pair of upper magnetic flux conductors defining between them an opening (50) into which the bottom end of the vessel containing the liquid suspension of magnetically attractable particles is inserted in order to form part-annular pellets comprising the magnetically attractable particles.

6. A method as claimed in any one of claims 1 to 5, wherein the magnet system comprises at least one permanent magnet.

7. A method as claimed in any one of claims 1 to 3, wherein the magnet system comprises a back flow plate (2) formed of magnetisable material; a plurality of pairs of permanent magnets (3) each having opposed first and second ends, the first end of each said magnet being positioned on the back flow plate with a N-S axis oriented perpendicular to the back flow plate, the polarity of each permanent magnet of a pair being opposite to that of its neighbour, the permanent magnets of a pair being spaced apart to define between them a work station in the form of an upwardly-open hole into which the bottom end of the vessel containing the liquid suspension of magnetically attractable particles is inserted in order to form an annular or part-annular pellets comprising the magnetically attractable particles.

8. A method as claimed in any one of claims 1 to 7, wherein sufficient fresh liquid is added to the vessel to submerge the annular or part-annular pellet.

9. A method as claimed in any one of claims 1 to 8, wherein the vessel is a round-bottom or conical-bottom vessel.

10. Apparatus which comprises:
an array of vessels (6, 7) each to contain a liquid suspension of magnetically attractable particles;
a pipetting system for transferring liquid to and from the array of vessels;
and a magnet system; and
means for movably mounting the magnet system relative to the array of vessels,
wherein the magnet system comprises: a back flow plate (2) formed of magnetisable material; a plurality of pairs of permanent magnets (3) each having opposed first and second ends, the first end of each said magnet being positioned on the back flow plate with a N-S axis oriented perpendicular to the back flow plate, the polarity of each permanent magnet of a pair being opposite to that of its neighbour, the permanent magnets of a pair being spaced apart to define between them a work station,
and wherein there are provided means for effecting vertical movement of the magnet system relative to the array of vessels said means being adapted to bring the magnet system to any one of three positions and hold the magnet system in that position, the positions being:
i) engaged with the array of vessels in an annular separation position;
ii) free of the array of vessels; and
iii) engaged with the array of vessels in a pull-down position.

11. Apparatus as claimed in claim 10, wherein the permanent magnets of a pair are spaced apart to define between them a workstation in the form of an upwardly-open hole for receiving a vessel.

## Patentansprüche

1. Magnetisches Trennverfahren, umfassend die Schritte:
a) Bringen eines Gefäßes mit einem Boden und mit einer flüssigen Suspension magnetisch anziehbarer Teilchen in die Nähe eines Magnetsystems durch Bewirken vertikaler Bewegung des Magnetsystems in bezug auf das Gefäß, wodurch nahe dem, aber nicht an dem Boden des Gefäßes ein kreisförmiges oder teilweise kreisförmiges, die magnetisch anziehbaren Teilchen umfassendes Pellet gebildet wird;
b) Entnahme der überstehenden Flüssigkeit aus dem Gefäß, und
c) Zugabe frischer Flüssigkeit zu dem Gefäß;
d) Bewegen desselben Magnetsystems in eine darunterliegende Position und Halten desselben in der Position, so daß die magnetisch anziehbaren Teilchen zum Boden des Gefäßes gezogen werden;
e) Entfernen des Magnetsystems;
f) und Resuspendieren der magnetisch anziehbaren Teilchen in der frischen Flüssigkeit.

2. Verfahren nach Anspruch 1, angewandt auf die Behandlung einer Polymerlösung in einem Gefäß mit einem Boden durch die Verwendung von magnetisch anziehbaren Teilchen, die nicht spezifisch das Polymer binden, umfassend die Schritte:
- Suspendieren der magnetisch anziehbaren Teilchen in der Lösung zur Bildung einer flüssigen Suspension der magnetisch anziehbaren Teilchen,
- Fällen des Polymers aus der Lösung, wodurch es unspezifisch mit den magnetisch anziehbaren Teilchen assoziiert wird,
- Ausführen von Schritten a), b) und c) nach Anspruch 1,
- und Wiederauflösen des Polymers in der frischen Flüssigkeit.

3. Verfahren nach Anspruch 2, wobei das Polymer eine Nucleinsäure ist.

4. Verfahren nach einem der Ansprüche 1 bis 3, wobei das Magnetsystem einen einzigen Magneten (30), kombiniert mit einem oben offenen zylindrischen Magnetflußleiter (32), umfaßt und der Boden des Gefäßes, der die flüssige Suspension der magnetisch anziehbaren Teilchen enthält, in das offene obere Ende des zylindrischen Magnetflußleiters eingesetzt wird, damit ein kreisförmiges Pellet, das die magnetisch anziehbaren Teilchen umfaßt, gebildet wird.

5. Verfahren nach einem der Ansprüche 1 bis 3, wobei das Magnetsystem ein Paar Magneten (40, 42) an gegenüberliegenden Seiten des die flüssige Suspension enthaltenden Gefäßes (die Position, die durch das Gefäß einzunehmen ist) umfaßt, wobei jeder Magnet mit einem unteren Pol zu einem gegenüberliegenden unteren Pol des anderen Magneten weisend geneigt angeordnet ist, die unteren Seitenflächen der Magneten durch einen unteren Magnetflußleiter verbunden sind, und wobei die obere Seitenfläche von jedem Magneten einen oberen Magnetflußleiter (46, 48) trägt, wobei das Paar oberer Magnetflußleiter zwischen ihnen eine Öffnung (50) festlegt, in die der Boden des die flüssige Suspension von magnetisch anziehbaren Teilchen enthaltenden Gefäßes eingesetzt wird, um teilweise kreisförmige Pellets, die die magnetisch anziehbaren Teilchen umfassen, auszubilden.

6. Verfahren nach einem der Ansprüche 1 bis 5, wobei das Magnetsystem mindestens einen Permanentmagneten umfaßt.

7. Verfahren nach einem der Ansprüche 1 bis 3, wobei das Magnetsystem umfaßt: eine Rückstromplatte (2), gebildet aus magnetisierbarem Material, eine Vielzahl von Paaren von Permanentmagneten (3), jeweils mit gegenüberliegenden ersten und zweiten Enden, wobei das erste Ende von jedem Magneten an der Rückstromplatte mit einer Nord-Süd-Achse senkrecht zu der Rückstromplatte orientiert angeordnet ist, die Polarität von jedem Permanentmagneten eines Paares zu jenem seines Nachbarn entgegengesetzt ist, wobei die Permanentmagneten eines Paares, zur Festlegung eines Arbeitsplatzes zwischen ihnen, in Form einer aufwärts offenen Öffnung, in die der Boden des die flüssige Suspension von magnetisch anziehbaren Teilchen enthaltenden Gefäßes zur Ausbildung eines kreisförmigen oder teilweise kreisförmigen, die magnetisch anziehbaren Teilchen umfassenden Pellets, eingesetzt wird, voneinander beabstandet sind.

8. Verfahren nach einem der Ansprüche 1 bis 7, wobei ausreichend frische Flüssigkeit zu dem Gefäß gegeben wird, um das kreisförmige oder teilweise kreisförmige Pellet zu überschichten.

9. Verfahren nach einem der Ansprüche 1 bis 8, wobei das Gefäß ein Gefäß mit rundem oder kegelförmigem Boden ist.

10. Vorrichtung, umfassend:
eine Anordnung von Gefäßen (5, 7), die jeweils eine flüssige Suspension von magnetisch anziehbaren Teilchen enthalten,
ein Pipettiersystem zur Überführung der Flüssigkeit zu und von der Anordnung von Gefäßen;
und ein Magnetsystem; und
eine Vorrichtung zu einer in bezug auf die Anordnung von Gefäßen beweglichen Befestigung des Magnetsystems,
wobei das Magnetsystem umfaßt: eIne Rückstromplatte (2), gebildet aus magnetisierbarem Material, eine Vielzahl von Paaren von Permanentmagneten (3), die jeweils gegenüberliegende erste und zweite Enden aufweisen, wobei das erste Ende von jedem der Magneten an der Rückstromplatte mit einer Nord-Süd-Achse senkrecht zur Rückstromplatte orientiert angeordnet ist, wobei die Polarität von jedem Permanentmagneten eines Paares zu jener seines Nachbarn entgegengesetzt ist, wobei die Permanentmagneten eines Paares, zur Festlegung eines Arbeitsplatzes zwischen ihnen voneinander beabstandet sind,
und wobei eine Vorrichtung zum Bewirken einer vertikalen Bewegung des Magnetsystems in bezug auf die Anordnung von Gefäßen bereitgestellt wird, wobei die Vorrichtung so ausgelegt ist, daß das Magnetsystem in eine beliebige der drei Positionen gebracht wird und das Magnetsystem in der Position hält, wobei die Positionen sind:
i) im Verbund mit der Anordnung von Gefäßen in einer kreisförmigen Trennposition;
ii) ohne die Anordnung von Gefäßen; und
iii) im Verbund mit der Anordnung von Gefäßen in einer herabgezogenen Position.

11. Vorrichtung nach Anspruch 10, wobei zur Aufnahme eines Gefäßes die Permanentmagneten eines Paares in Form einer aufwärts offenen Öffnung zur Festlegung eines Arbeitsplatzes zwischen ihnen voneinander beabstandet sind.

## Revendications

1. Procédé de séparation magnétique qui comprend les étapes consistant:
a) à amener un récipient ayant une extrémité inférieure et contenant une suspension liquide de particules pouvant être attirées magnétiquement à proximité d'un système à aimant, en effectuant un mouvement vertical du système à aimant par rapport au récipient, ainsi il se forme un culot annulaire ou partiellement annulaire comprenant les particules pouvant être attirées magnétiquement près de, mais pas à, l'extrémité inférieure du récipient,
b) à retirer le liquide surnageant du récipient, et
c) à ajouter un liquide frais au récipient;
d) à déplacer le même système à aimant vers une position abaissée et à le maintenir dans cette position de façon à attirer les particules pouvant être attirées magnétiquement vers le fond du récipient;
e) à retirer le système à aimant;
f) et à remettre en suspension les particules pouvant être attirées magnétiquement dans le liquide frais.

2. Procédé selon la revendication 1, appliqué au traitement d'une solution d'un polymère dans un récipient ayant une extrémité inférieure, en utilisant des particules pouvant être attirées magnétiquement qui ne se lient pas spécifiquement au polymère, comprenant les étapes consistant:
- à mettre en suspension les particules pouvant être attirées magnétiquement dans la solution de façon à former une suspension liquide de particules pouvant être attirées magnétiquement,
- à précipiter le polymère de la solution, ainsi il devient non spécifiquement associé aux particules pouvant être attirées magnétiquement,
- à effectuer les étapes a), b) et c) de la revendication 1,
- et à redissoudre le polymère dans le liquide frais.

3. Procédé selon la revendication 2, dans lequel le polymère est un acide nucléique.

4. Procédé selon l'une quelconque des revendications 1 à 3, dans lequel le système à aimant comprend un seul aimant (30), combiné avec un conducteur de flux magnétique cylindrique à ouverture supérieure (32), et l'extrémité inférieure du récipient contenant la suspension liquide de particules pouvant être attirées magnétiquement est insérée dans l'ouverture supérieure du conducteur de flux magnétique cylindrique afin de former un culot annulaire comprenant les particules pouvant être attirées magnétiquement.

5. Procédé selon l'une quelconque des revendications 1 à 3, dans lequel le système à aimant comprend une paire d'aimants (40, 42) sur les côtés opposés du (position à occuper par le) récipient contenant la suspension liquide, chaque aimant étant incliné avec un pôle inférieur en face d'un pôle opposé inférieur de l'autre aimant, les faces latérales inférieures des aimants étant reliées ensemble par un conducteur de flux magnétique inférieur, et dans lequel la face latérale supérieure de chaque aimant supporte un conducteur de flux magnétique supérieur (46, 48), la paire de conducteurs de flux magnétique supérieurs définissant entre eux une ouverture (50) dans laquelle on insère l'extrémité inférieure du récipient contenant la suspension liquide de particules pouvant être attirées magnétiquement afin de former des culots partiellement annulaires comprenant les particules pouvant être attirées magnétiquement.

6. Procédé selon l'une quelconque des revendications 1 à 5, dans lequel le système à aimant comprend au moins un aimant permanent.

7. Procédé selon l'une quelconque des revendications 1 à 3, dans lequel le système à aimant comprend une plaque de retour de flux (2) constituée de matière magnétisable; une pluralité de paires d'aimants permanents (3) chacun ayant des première et seconde extrémités opposées, la première extrémité de chaque dit aimant étant placée sur la plaque de retour de flux avec un axe N-S orienté perpendiculaire à la plaque de retour de flux, la polarité de chaque aimant permanent d'une paire étant opposée à celle de son voisin, les aimants permanents d'une paire étant espacés l'un de l'autre pour définir entre eux une station de travail sous la forme d'un trou à ouverture par le haut dans lequel on insère l'extrémité inférieure du récipient contenant la suspension liquide de particules pouvant être attirées magnétiquement afin de former un culot annulaire ou partiellement annulaire comprenant les particules pouvant être attirées magnétiquement.

8. Procédé selon l'une quelconque des revendications 1 à 7, dans lequel on ajoute suffisamment de liquide frais au récipient pour submerger le culot annulaire ou partiellement annulaire.

9. Procédé selon l'une quelconque des revendications 1 à 8, dans lequel le récipient est un récipient à fond rond ou à fond conique.

10. Appareil qui comprend:
une matrice de récipients (6, 7) pour contenir chacun une suspension liquide de particules pouvant être attirées magnétiquement;
un système de pipetage pour transférer un liquide vers ou depuis la matrice de récipients;
et un système à aimant; et
des moyens pour monter de manière amovible le système à aimant par rapport à la matrice de récipients,
dans lequel le système à aimant comprend: une plaque de retour de flux (2) constituée de matière magnétisable; une pluralité de paires d'aimants permanents (3) ayant chacun des première et seconde extrémités opposées, la première extrémité de chaque dit aimant étant placée sur la plaque de retour de flux avec un axe N-S orienté perpendiculaire à la plaque de retour de flux, la polarité de chaque aimant permanent d'une paire étant opposée à celle de son voisin, les aimants permanents d'une paire étant espacés l'un de l'autre pour définir entre eux une station de travail,
et dans lequel on a prévu des moyens pour effectuer un mouvement vertical du système à aimant par rapport à la matrice de récipients lesdits moyens étant conçus pour amener le système à aimant dans l'une quelconque des trois positions et maintenir le système à aimant dans cette position, les positions étant:
i) en prise avec la matrice de récipients dans une position de séparation annulaire;
ii) sans prise avec la matrice de récipients; et
iii) en prise avec la matrice de récipients dans une position descendante.

11. Appareil selon la revendication 10, dans lequel les aimants permanents d'une paire sont espacés l'un de l'autre pour définir entre eux une station de travail sous la forme d'un trou à ouverture par le haut pour recevoir un récipient.
